# EUROPEAN PATENT APPLICATION

(11) **EP 3 567 529 A1**
(43) Date of publication of application: **13.11.2019**
(21) Application number: 18736029.2
(22) Date of filing: 09.01.2018
(51) Int. Cl.: G06N 3/08, G06N 3/04, G06N 99/00

(54) **SENSING SYSTEM AND SENSING METHOD USING MACHINE LEARNING**

(30) Priority: 09.01.2017 KR 20170002695; 31.03.2017 KR 20170041405
(71) Applicant: Industry-Academia Cooperation Group of Sejong University, Seoul 05006 (KR)
(72) Inventor: SOHN, Kee-sun, Seoul 06057 (KR); KIM, Ji-sik, Busan 46230 (KR); SOHN, Kee-min, Seoul 06003 (KR)
(74) Representative: Beck & Rössig European Patent Attorneys
(86) International application number: PCT/KR2018/000383
(87) International publication number: WO 2018/128513

(57) **Abstract**

The present invention relates to a sensing method using machine learning. A sensing method according to the present invention comprises the steps of: providing a stimulus multiple times to a random position on a material for generating a detectable signal in response to an external stimulus; performing machine learning by using the signal generated through the stimuli; and predicting a location or degree of a stimulus which is provided to the material, by using a function derived through the machine learning.

## Description

### Technical Field

The present invention relates to a sensing method using machine learning and a sensing system.

### Background Art

Ceaseless efforts to manufacture artificial skin, called electronic skin, have been made, and in terms of functional performance, the artificial skin has become similar to actual human skin.

A main factor that affects performance of the electronic skin is tactile sensing including recognition of a contact position and strain (or pressure) of the contact position.

A plurality of strain sensors has been developed for such electronic skin. Even allowing for any type of strain sensor used in the conventional electronic skin, a common configuration provided in all types of electronic skin is a regular arrangement of device elements called a pattern with respect to a so-called specific region.

Such a pattern, i.e., a multimode micro-(or macro-) element arrangement on a single layer or multiple layers, is essential for implementing reliable electronic skin, and without the multimode arrangement, there is no way to systematically detect a pressure distribution in a specific region of skin.

In this regard, various types of tactile or position sensors, such as resistive, capacitive, inductive, piezoresistive, optical, self-molding, binary, piezoelectric, and hydraulic type sensor devices, have been developed.

However, all the sensors have a specific pattern of device elements on a multilayer substrate with a logic circuit design for processing detected signals. For example, there are various patterns ranging from an extremely simple spacer pattern, which is disposed between layers developed for resistive touch panels, to an extremely complicated pattern such as a strain gauge pattern with a complicated wire arrangement or a thin-film transistor (TFT) array pattern.

In addition, there are various pattern manufacturing methods ranging from a simple printing method to a method using a complicated semiconductor process. However, in manufacturing a reliable electronic skin at low costs, a pattern forming process has become one of the main barriers.

Furthermore, in the conventional device with respect to electronic skin, a position of a load may be recognized, but there is a problem in that a level of pressure of the load is difficult to recognize.

### Disclosure

### Technical Problem

The present invention is directed to providing a sensing method using machine learning technique, which is usable for various devices including electronic skin to recognize a position of a load and accurately measure a degree of the load, and a sensing system to which the sensing method is applied.

In addition, the present invention is directed at providing a sensing method using machine learning technique, which is capable of recognizing a position of a load and measuring a degree of the load even when a crude, raw material on which a pattern is not formed is used.

### Technical Solution

To solve the above problem, a sensing method according to the present invention includes applying a stimulus multiple times to random positions at a material, recording a set of exact position and load values along with a signal generated in response to the stimulus (the signal is detectable from several probe terminals located at the edge of the material), performing machine learning using the recorded data, and finally enabling the prediction of a position and a degree of the stimulus applied to the material using the model function derived through the machine learning.

In the sensing method, the material configured to generate the signal that is detectable may be divided into a plurality of virtual sectors, the stimulus may be applied to each sector multiple times, and the machine learning may be performed using a signal generated by the stimulus on each sector.

In the sensing method, the machine learning may be performed using coordinates of the position, to which the stimulus is applied, together with the generated signal.

In the sensing method, in terms of simplification, the material may be a material on which a regular pattern is not formed. However, a material on which a certain pattern is formed is not limited in terms of a function.

In the sensing method, the sensing method may be used for electronic skin, a touch panel, a flexible keyboard, a sign language interpretation glove, a safety diagnosis of a social infrastructure, a diagnosis of motility and motility disorder diagnosis in a gastrointestinal tract, or a sensor for a large area strain gauge.

In the sensing method, when the stimulus is applied by dividing the material into the plurality of virtual sectors, a vertical load may be applied to each of the sectors or multiple loads including any types of complex loads such as tearing, shearing, rubbing, pinching, etc. may be applied to two or more of the sectors.

In the sensing method, even when the stimulus is applied to a random position without dividing the material into the plurality of virtual sectors, a single vertical load may be applied, or vertical loads may be concurrently applied to several positions, or multiple, complex loads such as tearing, shearing, rubbing, pinching, etc. may be applied to a certain area.

In order to solve the above other problems, a sensing system according to the present invention includes a material configured to generate a signal that is detectable in response to a stimulus from an outside, a storage unit configured to store a function derived by performing machine learning using signals generated by applying the stimulus to the material multiple times, and an operation unit configured to operate a position or a degree of the stimulus applied to the material using the function.

In the sensing system, the material configured to generate the signal that is detectable may be divided into a plurality of virtual sectors, the stimulus may be applied to each sector multiple times, and the machine learning may be performed using a signal generated in each sector as well as the recorded position and load data.

In the sensing system, the machine learning may be performed using coordinates of the position, to which the stimulus is applied, together with the generated signal.

In the sensing system, in terms of simplification, the material may be a material on which a regular pattern is not formed. The invented sensing system is not a device but a just crude, raw material. However, a material on which a certain pattern is formed is not limited in terms of a function.

The sensing system may be used for electronic skin, a touch panel, a flexible keyboard, a sign language interpretation glove, a safety diagnosis of a social infrastructure, a diagnosis of motility and motility disorder diagnosis in a gastrointestinal tract, or a sensor for a large area strain gauge.

### Advantageous Effects

According to a sensing method and a sensing system based on the present invention, a position and a degree of load applied to the sensing system can be accurately measured from the electrical signal that is detectable from several probe terminals located at the edge of the material by the assistance of the machine learning.

In addition, according to an exemplary embodiment of the present invention, a position and a degree of load applied to the sensing system can be accurately predicted without a regular device element pattern, thereby significantly reducing manufacturing costs of a field such as artificial skin.

Furthermore, according to an exemplary embodiment of the present invention, when the standardization of a material is performed to some extent, a learning result of a specific material can be applied to other materials of the same kind, thereby considerably reducing learning time and cost.

In addition, according to an exemplary embodiment of the present invention, by using a simple sheet, patch, or film such as a piezoresistive composite patch including multi-walled carbon nanotubes (CNTs) uniformly distributed in a polydimethylsiloxane (PDMS) film or patch, a large area can be implemented and high reliable strain (and a position) can be sensed while not requiring a complex, high-cost fabrication process involving regular patterns or a multilayered structure.

Furthermore, according to an exemplary embodiment of the present invention, pressure can be sensed in a wide range from a low touch level to a level of feeling pain by controlling a fraction of a curing agent and an addition ratio of CNTs in a PDMS substrate, a thickness of a sheet, and the like.

In addition, according to an exemplary embodiment of the present invention, even when a complicated multiple load is applied to two or more sectors other than a simple vertical load applied to a single sector, sensing is possible through deep neural network (DNN), convolutional neural network (CNN), or recurrent neural network (RNN) learning.

Furthermore, a sensing method and a sensing system according to the present invention can be applied to electronic skin, a touch panel, a flexible keyboard, a sign language interpretation glove, a safety diagnosis of a social infrastructure, a diagnosis of motility and motility disorder diagnosis in a gastrointestinal tract, or a sensor for a large area strain gauge.

For example, when a keyboard is printed on a surface of a material including a very thin flexible material (for example, a piezoresistive composite film or patch) configured to generate a detectable signal and is subjected to a learning process according to the present invention, the keyboard can be used as a flexible keyboard. The keyboard can be easily portable, thereby providing improved convenience as compared with the conventional portable keyboards.

### Description of Drawings

FIG. 1 is a schematic diagram illustrating a multi-walled carbon nanotube (CNT)/polydimethylsiloxane (PDMS) composite film used as electronic skin in an exemplary embodiment of the present invention.
FIG. 2 shows an arrangement of a device (wooden bar and toy finger controlled by an actuator) configured to apply a load to a CNT/PDMS composite film for machine learning of electronic skin and terminals (see a center image) configured to sense an electrical signal according to an exemplary embodiment of the present invention.
FIG. 3 is a configuration diagram illustrating a learning and testing system of electric skin according to an exemplary embodiment.
FIG. 4A shows a plot showing a load (displacement or pressure) and electrical resistance as a time function with respect to periodic load with a frequency of 1 Hz applied to a uniaxial tensile test specimen shown at a right side of the drawing.
FIG. 4B shows a plot of electrical resistance with respect to displacement at frequencies of 1 Hz, 3 Hz, and 6 Hz of the uniaxial tensile specimen.
FIG. 4C shows displacement (pressure) and a pressure versus displacement curve (see a lower right side) in a pressure range of 0 Mpa to 1.2 MPa at positions of 6×6 sectors with respect to electrode signals of 8 probe terminals.
FIG. 5 is a hierarchy diagram illustrating deep learning applied to an exemplary embodiment of the present invention.
FIG. 6 shows data set structures used in an exemplary embodiment of the present invention.
FIG. 7A shows deep neural network (DNN) architectures for position recognition with 4 features, 8 features, and 16 features used in an exemplary embodiment of the present invention.
FIG. 7B shows DNN architectures for pressure recognition with 8 features and 16 features used in an exemplary embodiment of the present invention.
FIG. 8 shows a regression-type DNN architecture for obtaining actual coordinates without division into virtual sectors.
FIG. 9 shows a plot of measured displacement (pressure) with respect to predicted displacement (pressure) for each of 36 (6×6) sectors performed in an exemplary embodiment.
FIG. 10 shows a plot of test pressure with respect to predicted pressure for each of 36 sectors performed in an exemplary embodiment.
FIG. 11 shows eight electrical resistance curves as a function of pressure at a specific sector position (fourth row and third column of a 6×6 sector matrix) of each of a thick and rigid sheet (see FIG. 11A) for high pressure measurement and a thin and smooth film for low pressure sensing (see FIG. 11B).

### Modes of the Invention

Hereinafter, exemplary embodiments of the present invention will be described. However, the present invention should not be construed as being limited to the following exemplary embodiments. Rather, the exemplary embodiments should be construed as being embodied in many different forms without departing from the scope of the present invention. Although the materials adopted in the exemplary embodiments of the present invention were limited to CNT-PDMS composite, a variety of choices should be available, e.g., metal powder or flake, graphene, or any other conducting micro-(or nano-) materials for CNT, and PMMA, Ecoflex, or any other flexible matrix material for PDMS.

### Manufacture of CNT/PDMS Piezoresistive Composite Film

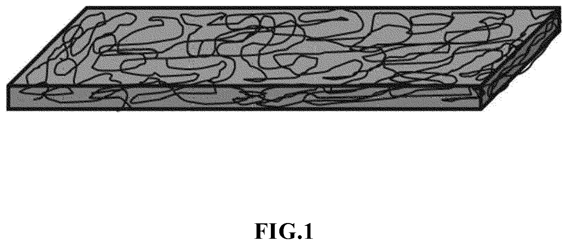

As schematically shown in FIG. 1, a composite in which multi-walled carbon nanotubes (MWCNTs (hereinafter, referred to as CNTs)) are randomly distributed was synthesized using commercial CNTs (manufactured by Nano-Materials Technology Co.) and polydimethylsiloxane (PDMS: Sylgard® 184 Silicone Elastomer).

CNTs having a multi-wall with a length of 5 µm and a diameter of 20 nm were used. Liquefied PDMS was introduced into a plastic cylinder container, and 1 wt% of the CNTs were mixed with the PDMS and were stirred. Then, the PDMS was cured to manufacture a piezoresistive patch.

Specifically, in order for the CNTs to be uniformly dispersed without being aggregated in the PDMS, a small amount of alumina balls having a diameter of 10 mm was introduced and stirred at a rate of 400 rpm for 2 hours using a planetary shear mixer. A PDMS curing agent was added in an amount of 10% of the weight of the PDMS and was maintained in the planetary shear mixer for 20 minutes. Finally, degassing treatment was performed for 20 minutes in a vacuum state to prevent bubbles from being trapped in the composite. A mold was prepared on a glass substrate, and a piezoresistive CNT/PDMS composite was molded using a doctor blade technique. The glass substrate was heated at a temperature of 60 °C for 30 minutes to solidify the CNT/PDMS composite so as to have dimensions of 40 mm width×40 mm length×5 mm thickness.

In addition, other molds having depths of 0.1 mm, 1 mm, and 2 mm were also used to form a thin film, and elastic properties of a sheet were diversified by adjusting the PDMS curing agent.

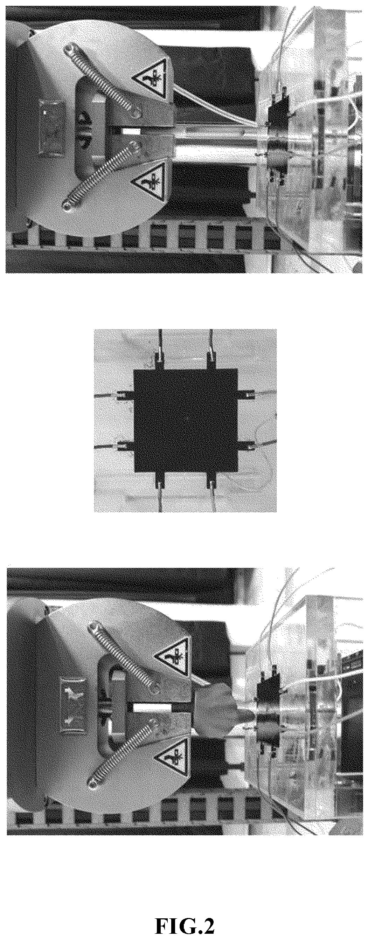

As confirmed in a center image of FIG. 2, a CNT-PDMS composite sheet, in which any pattern structure is not formed on a surface thereof, was manufactured through such a process.

Although the CNT-PDMS composite sheet was used in an exemplary embodiment of the present invention, sheets made of various materials, such as an indium tin oxide (ITO) substrate and a rubber plate in which a conductive material is provided so as to not form a pattern, may be used as long as the sheets may generate a signal in response to a stimulus.

### Collection of Learning and Test Data

As shown in the center image of FIG. 2, several copper wires were connected to CNT-PDMS terminals arranged at intervals of 20 mm on sides of a CNT-PDMS patch. In addition, a ground was disposed at a center of the CNT-PDMS patch, and a direct current (DC) bias voltage of 5V was applied.

Since various resistance measurement probe terminals play an important role in a sensor patch having no pattern, one to four probes were variously used on each side of the sensor patch. FIG. 2 is an actual image of a patch (see the center image) in which two probes are connected to each side thereof in an exemplary embodiment of the present invention.

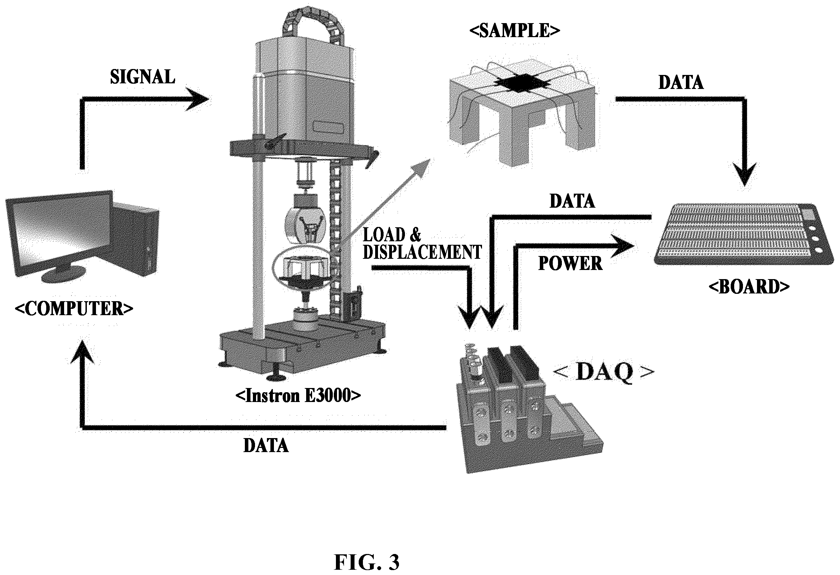

In addition, a configuration diagram of a test operation is shown in FIG. 3. As shown in FIG. 3, learning and testing for electronic skin according to an exemplary embodiment of the present invention were performed using a device (Instron E3000) configured to apply a load, a specimen mounted on the device, a probe configured to detect an electrical signal from the specimen, data acquisition (DAQ) configured to convert the load and displacement data received from the device and electrical signal data received from the probe through a circuit into digital data, and a computer configured to operate the converted data and transfer a signal to the device.

Patch learning was performed using Instron E3000. An entire region of a piezoresistive patch was virtually divided into sectors having an area of m×m (m=4, 6, and 10). A single sector area (i.e., spatial resolution) corresponding to a finger contact area is provided through such virtual sector division.

As shown at a right side of FIG. 2, a cylindrical bar with a rounded tip was mounted in the Instron E3000 to systematically press a selected sector region of the piezoresistive patch.

In addition, in order to change a pressing point, the piezoresistive patch was disposed on a movable x-y stage.

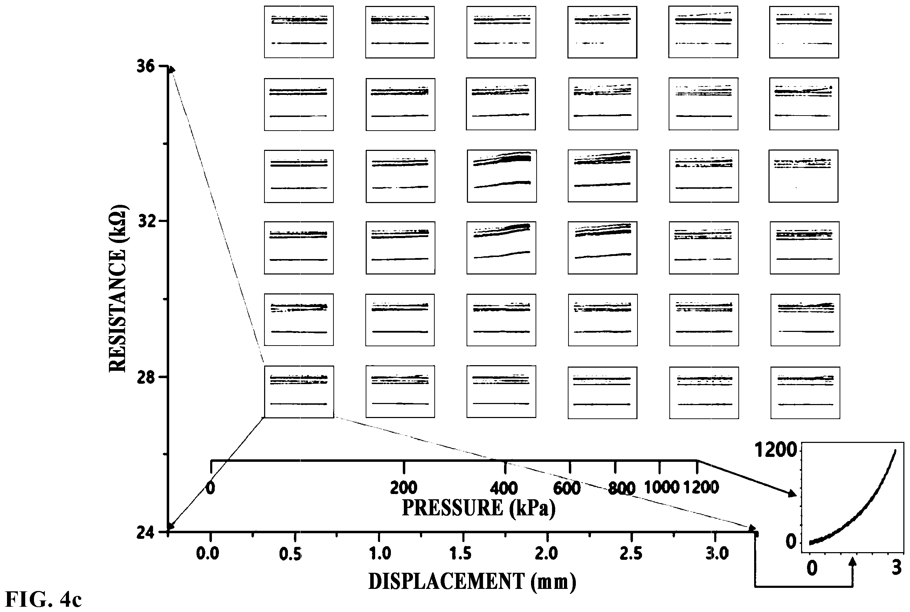

As shown in FIG. 4C, pieces of data on electrical resistance were collected from 4, 8, and 16 probes disposed on the sides of the patch by repeatedly pressing each sector. In combination with the collection of training data sets through such a process, test data sets were separately collected for training data using an artificial plastic finger which is softer than a stick and is more similar to a human finger.

### Configuration of Deep Neural Network (DNN)

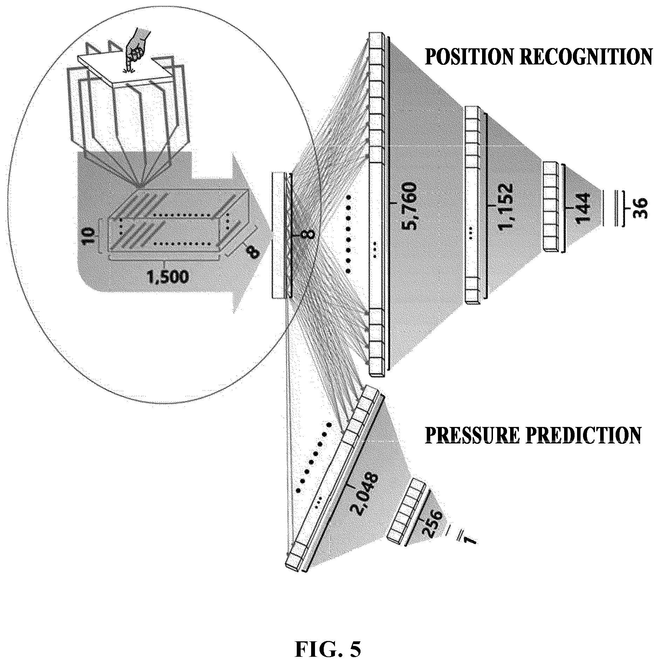

Basically, a DNN of the present exemplary embodiment includes two channels as shown in FIG. 5. One channel is for recognizing a position, and the other channel is for recognizing strain (pressure).

The two channels are simultaneously operated in a real sensing situation. Input data is vectorized into an n-dimensional (1×n) vector, and elements of the vector mean the number of actual resistance values measured from each of n terminal electrodes (n=4, 8, and 16). The number n of elements of an input vector, i.e., dimensions of the input vector, may be expressed by the number of input features (= the number of probe terminals for the electrical resistance measurement).

A specific level of pressure (0 MPa to 1.2 MPa) was applied to a relevant sector, and data was collected during pressing. As a result, in the present exemplary embodiment, 1500 n-dimensional input vectors were collected per each sector, and independent measurement was performed 10 times in each sector. Thus, total training data sets included 540,000 (1,500×36×10) n-dimensional input vectors for 6×6 sectors.

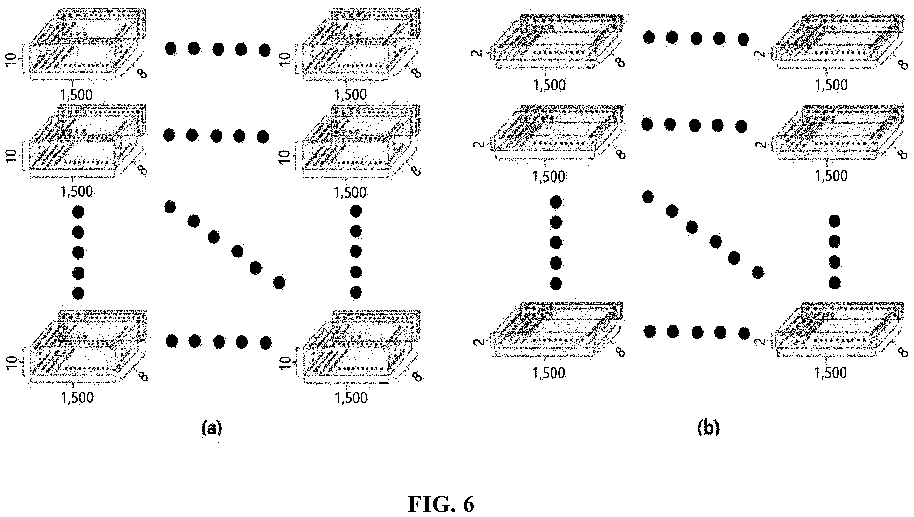

A training data set structure and a test data set structure of the present exemplary embodiment were schematically shown in FIGS. 6A and 6B. Label (output) data for position recognition indicates sector coordinates indicating rows and columns of a 6×6 matrix, and label data for strain (pressure) indicates an actual number. A test data set has a similar data structure, but only independent measurement was performed twice in each sector. Most notably, the test data set was independently prepared using a toy finger that was different from a wooden bar used in the training data set of each sector.

It has been proven that piezoresistance of a CNT-PDMS material was not affected by a strain rate. This means that piezoresistance was only affected by an instantaneous state of a distribution of CNTs, which are conductors, a PDMS substrate, which is an insulator, and was not affected by a strain rate.

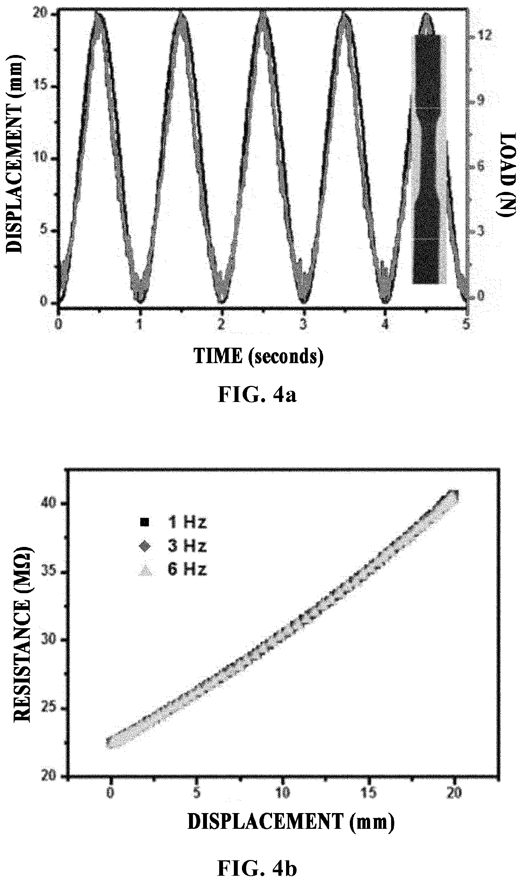

As shown in FIGS. 4A and 4B, it had been proven that piezoresistance had a complete linear relationship with strain (displacement or pressure) regardless of a load frequency.

In the present invention, the expression "pressure" may be used together with "strain" and "displacement." The terms "pressure," "displacement," and "strain" should be treated equally as an output signal from a practical point of view. This is because the terms have a perfect linear relationship in static and dynamic conditions without inelastic behavior or plastic behavior. As a result, there was no difference in terms used herein.

In an exemplary embodiment of the present invention, a DNN architecture is characterized by the number of intermediate (hidden) layers, the number of nodes in each layer (neurons), detailed contents of an activation function, a rectified linear unit (ReLU), a batch size, an epoch control, and validation test setting, and the like.

The number of nodes in an input layer is equal to each of the number of features, dimensions of an input vector, and the number of probe terminal electrodes.

A final architecture, i.e., the number of the intermediate (hidden) layers, the DNN architecture parameterized due to the adoption of the ReLU, and the number of the nodes in each layer have been determined through many attempts.

In an exemplary embodiment of the present invention, a process of deriving learning and testing results in detail was proposed only for a DNN architecture in which an input layer has 8 nodes and an output layer has 36 (6×6) nodes. Other DNN architectures in which input layers have 16 nodes and four nodes, and learning and testing results thereof are summarized in Table 1.

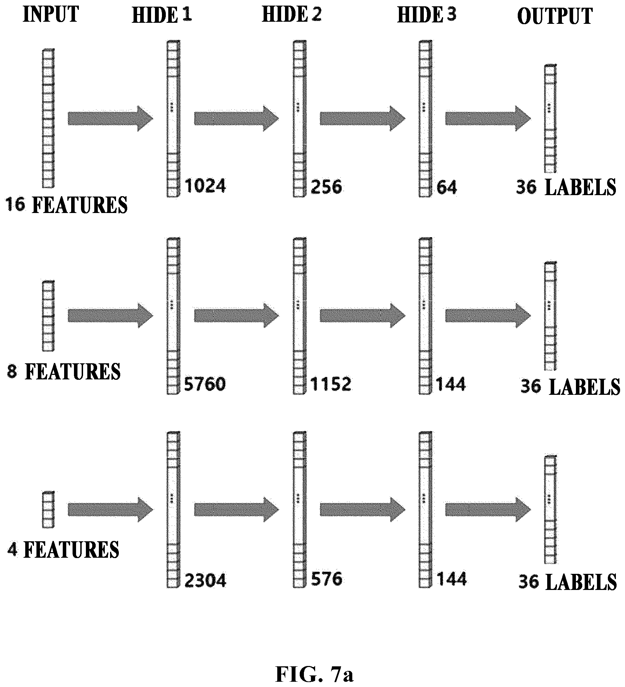

DNN architecture versions are summarized in Table 1 below along with learning and test results thereof, and schematic diagrams of DNN architectures with 4, 8, and 16 nodes are shown in FIGS. 7A (position recognition) and 7B (pressure recognition).

In the case of 16 nodes being used, slightly higher accuracy may be obtained for learning and testing. Fewer features (fewer probe terminal electrodes in an exemplary embodiment of the present invention) may be advantageous for the sake of simplicity such that a device including 8 probe terminal electrodes may be more preferable than a device including 16 electrodes. On the other hand, in the case of having four nodes, accuracy of learning and testing may be considerably lowered, and thus, it may be difficult to accurately predict position recognition.

Therefore, in the exemplary embodiment of the present invention, 8 nodes were used for electronic skin having no pattern in a 16-cm² region for optimization between the number of electrodes (system complexity) and accuracy.

In addition, data sets were also generated for DNNs with 4 features, 8 features, and 16 features (i.e., 4, 8, and 16 probe terminals), and 16 labels (i.e., a case of 4×4 sector division). Of course, since accuracy of learning and testing was considerably higher as compared with 6×6 sectors, results thereof were omitted.

A finer sector division is preferable for implementing a more delicate sensor, and data acquired through division into 6×6 sectors allows electronic skin to be more realistically simulated to be closer to actual skin.

### DNN for Position Recognition

For the above-mentioned reasons, in the exemplary embodiment of the present invention, electric resistance signals collected from 8 probe terminals were used as an 8-dimensional input vector in a DNN for position recognition by focusing on a DNN with 8 features and 36 labels.

As shown in FIG. 5, a DNN architecture for position recognition includes a total of four connected layers including an input layer and an output layer.

Each layer has 8 nodes, 5,760 nodes, 1,152 nodes, 144 nodes, and 36 nodes in order from the input layer to the output layer. A simple linear activation function was applied to a third layer from the input layer, but a softmax function as an activation function was applied to a finally connected layer. A rectified linear unit (ReLU) for applying an activation function to three hidden layers so as to have a threshold value of a max (0, x) was adopted for the three hidden layers, and a dropout of 30% was introduced. Validation was performed every 100 generations at a validation ratio of 0.25.

The learning accuracy was 98.97% based on individual training data and was 100% when all pieces of data were bound together in a group with respect to a specific sector (a data set consisting of 1,500 input vectors with the same label was considered the group, representing a single touch). An accuracy of 100% refers to a probability that a label most frequently matching a data set consisting of 1,500 input vectors is actually a specific label.

That is, a DNN model according to the exemplary embodiment of the present invention could precisely predict labels of 360 training data set groups without a single failure. Since 360 training data set groups represented 360 independent touches, all of the touches were successfully recognized by a DNN.

In the conventional DNN setups, in general, training data was commonly separated and used for testing. However, in the present exemplary embodiment, instead of the following such a criterion, a test data set was separately prepared using a completely different tool (toy finger) in order to secure robustness of a DNN.

The test data set was an 8-dimensional vector together with a corresponding label specifying a sector under pressure. A size of the test data set was smaller than a size of a training data set, and the test data set included 108,000 (1,500×36×2) input vectors with labels. Such testing was also performed based on individual data and group data.

In addition, in the present exemplary embodiment, 20% of the training data set for testing was separately set to perform typical training and testing. Results of the typical testing showed an accuracy of 99.78% based on the individual data and an accuracy of 100% based on the group data. This means that an accurate touch point was found at an accuracy of 99.78% and a time resolution of about 10⁻⁴ seconds every minute from eight electrical resistance values which are instantaneously sensed. In addition, accuracy of the typical case of a group data-based test was 100%.

Accuracy of an individual data-based test using a toy finger was 96.19%, and accuracy of a group data-based test using the toy finger was 97.22%.

The accuracy of the group data-based test resulted from 72 random touch tests, which generated a data set specifying a sector under pressure. To this end, 1,500 input vectors in group should be tested in the data set. The most frequently matching label was considered a correct sector indicated by group data. The accuracy of the group data-based test with respect to position recognition was 97.22%, which means that only two data sets of the 72 data sets are missed, i.e., the DNN incorrectly recognized only two touches of 72 touches.

However, the incorrectly recognized two touches were adjacent to a correct position. Since spatial resolution of skin proposed in the exemplary embodiment was about 6.6 mm, which was much smaller than a fingertip contact area, such a type of error may also occur in real skin.

As shown in Table 1 below, since an accuracy of 100% was obtained in a test for 16 probe terminal electrodes, a small error of touch position recognition in the above-described exemplary embodiment (using 8 probe terminal electrodes) was affected by the number of sensing terminals. Thus, the small error may not be very significant. In the case of using 16 probe terminal electrodes, accuracy of an individual data-based test was 99.28%, and accuracy of a group data-based test was 100%. That is, all of the touches were correctly recognized on a 16-feature DNN model.

On the other hand, since the above-described 8-feature DNN model is properly operated and a result thereof is acceptable, the 8-feature DNN model may be more suitable in terms of simplification of a system.

**Table 1**

| All sheets (virtually divided into 6×6 sectors and having spatial resolution of 6.6 m) | Position recognition | DNN architecture (Number of nodes in each layer) | | |
|---|---|---|---|---|
| | | Accuracy (%) of individual-based learning | | Accuracy (%) of individual-based learning |
| | | Accuracy (%) of group-based learning | | Accuracy (%) of group-based learning |
| | Pressure sensing | DNN architecture (Number of nodes in each layer) | | |
| | | %RMSE | | MAE |
| 16 probe terminal electrodes | Position recognition | 16-1,024-256-64-36 | | |
| | | 99.96 | | 99.28 |
| | | 100 | | 100 |
| | Pressure sensing | 16-4,096-1,024-128-1 | | |
| | | 3.96 | 0.053 | |
| 8 probe terminal electrodes | Position recognition | 8-5,760-1,152-144-36 | | |
| | | 98.97 (89.16) | | 96.19 (85.54) |
| | | 100 (99.72) | | 97.22 (100) |
| | Pressure sensing | 8-2048-256-1 | | |
| | | 3.12 (3.49) | 0.045 (0.030) | |
| 4 probe terminal electrodes | Position recognition | 4-2,304-576-144-36 | | |
| | | 87.87 | | 51.32 |
| | | 99.17 | | 50 |
| | Pressure sensing | - | | |

In addition, the present inventors have developed a regression-type DNN model for predicting an accurate touch point as an actual position coordinates in areal number together with a model for identifying (classifying) a position index through a sector number in a preset matrix as described above.

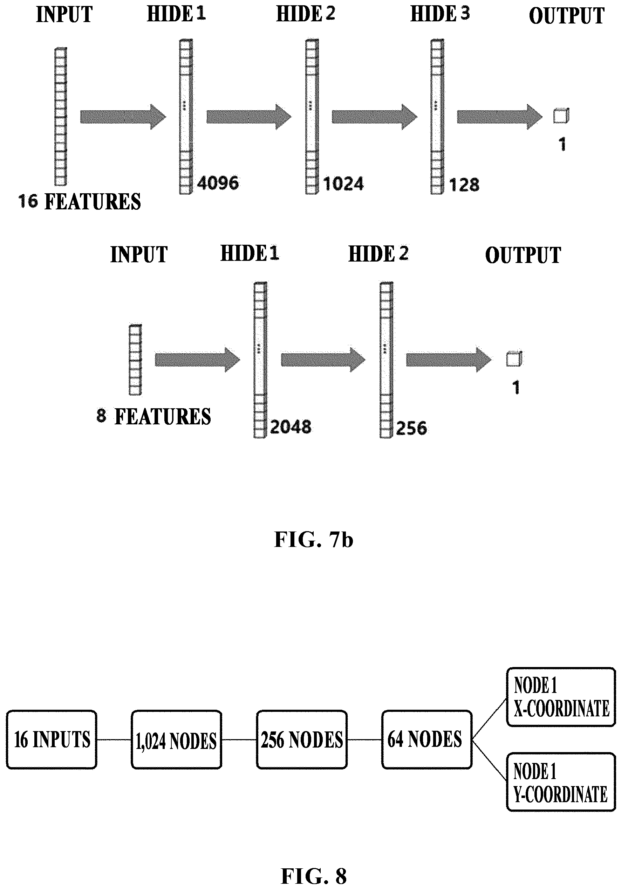

FIG. 8 illustrates an architecture of the regression-type DNN model.

In the case of using the regression-type DNN model, it is possible to obtain two values for specifying actual coordinates (X, Y) of a sheet region of electronic skin. Very high spatial resolution was obtained using the regression-type DNN model, and, as shown in Table 2, modeling was possible at a resolution of 0.78 mm ±0.44 mm using the regression-type DNN model.

**Table 2**

| | X-coordinate | Y-coordinate | Average |
|---|---|---|---|
| %RMSE | 3.24819833793 | 3.26769092788 | 3.2579446329 |
| RMSE | 0.113686941827 | 0.114369182476 | 0.114028062152 |
| MAE | 0.0848900556112 | 0.0943837681678 | 0.0896369118895 |
| Resolution | 0.78 mm ± 0.44 mm | | |

### DNN for Strain (Pressure) Evaluation

As shown in FIG. 5, another DNN for strain (pressure) evaluation was separately constructed with an architecture in which each layer has 8 nodes, 2,048 nodes, 256 nodes, and 1 node. Only a data set for a specific sector applied with pressure were independently used for training and testing of the DNN for the pressure evaluation, and repeated it for each of other sectors. This is because a correct position of the sector applied with pressure was specified at the beginning of a load using a position recognition DNN.

Accordingly, as shown in FIG. 5, training and testing were completed using 15,000 input vectors associated with corresponding pressure values as labels. An activation function for a hidden layer was a simple linear function, and an ReLU was applied like the case of the DNN for the position recognition. However, since an activation function of a final layer of the DNN for the pressure evaluation is not a softmax but a simple linear function, a final output value is an actual number representing displacement, which may be considered strain or pressure caused by a linear relationship.

Testing was also implemented using the toy finger data, which corresponds to 1,500 input vectors per sector and a total of 54,000 input vectors.

In the present exemplary embodiment, 1,500 test input vectors were randomly divided into 1,000 initial test data sets and 500 validation test data sets.

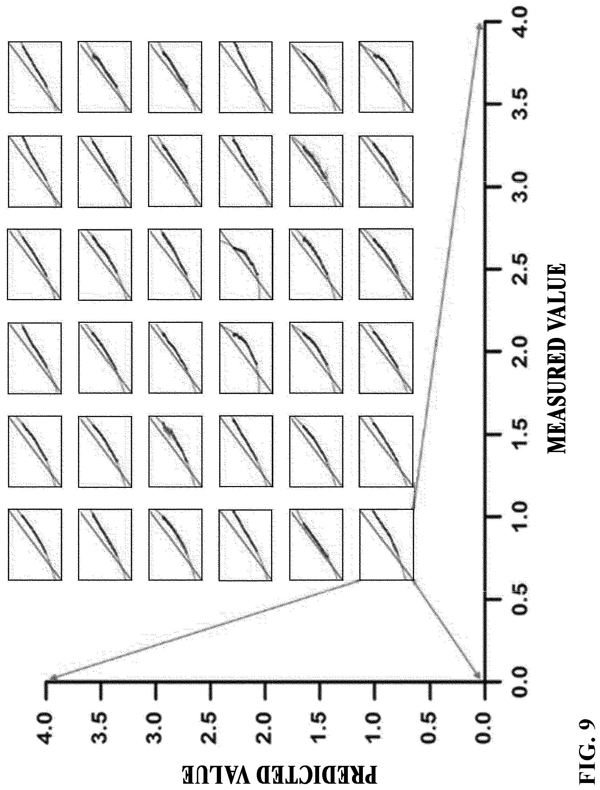

When the 1,000 initial test input vectors and corresponding pressure values were used in DNN testing, accuracy of the testing was unacceptable, and there was a discrepancy between a predicted value and a test value. A plot of measured displacement (pressure) with respect to predicted displacement (pressure) for each of the 36 sectors was shown in FIG. 7. The predicted pressure significantly deviated from test data, and a nonlinear curve deviating from a diagonal line was formed, as shown in FIG. 9. This deviation indicates not a scattered type but merely indicates a biased (deformed) feature.

This means that the deviation may be corrected through a simple data conversion. In this regard, in the present exemplary embodiment, a regression analysis was performed by introducing a second-order polynomial function as a regression model (ln(y)=β₀+β₁x+β₂x²), and results of the regression analysis were indicated by a red line in FIG. 9.

Thereafter, the DNN was tested using a test data set consisting of 500 input vectors and output pressure values, and data conversions of the regression analysis were integrated. Finally, in the present exemplary embodiment, a reliable predictive model for pressure recognition was obtained based on a DNN and a nonlinear regression.

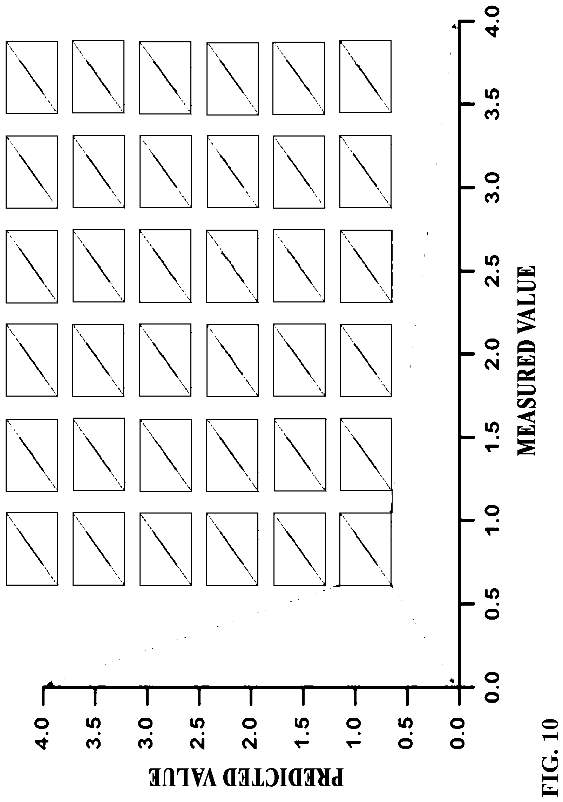

FIG. 10 shows a validation test data set showing a plot of a test input with respect to predicted pressure in each of the 36 sectors. In DNN modeling and a subsequent regression analysis, conversion of a test data set was made to achieve a complete linear relationship between experimentally measured pressure and predicted pressure. A pressure level could be immediately predicted at high accuracy at a position of a specific sector under pressure through such a calculation. An average RMSE with respect to 36 sectors is estimated at 3.12%, which is perfectly acceptable when a DNN is applied to real artificial skin.

### Spatial Resolution and Pressure Sensitivity

A spatial resolution of electronic skin for pressure sensing proposed in the exemplary embodiment of the present invention improves a function of a sensor. In the electronic skin of the present invention, spatial resolution may be very easily adjusted without an additional effort to physically change a structure of a device.

In the case of the electronic skin according to the exemplary embodiment of the present invention, spatial resolution may be adjusted by changing learning conditions, for example, by changing the number of features (the number of probe terminals), a virtual area dividing method, and amounts of data.

In the exemplary embodiment of the present invention, a spatial resolution of 6.6 mm was obtained with respect to 6×6 division electronic skin. This spatial resolution may be further lowered by adopting finer virtual subdivision and increasing the number of probe terminals. To this end, there is no need to change a structure of the electronic skin itself.

For example, when an electronic skin sheet having a size of 40×40 mm is learned using 10×10 virtual sectors and 16 probe terminals, spatial resolution may be lowered to about 4 mm, which is a very excellent result considering that spatial resolution of a high-tech tactile sensor is about 2.5 mm.

In the present invention, the degree of freedom of a virtual subdivision design is basically infinite so that any resolution may be achieved. However, a pick-block type test of the exemplary embodiment of the present invention was performed at limited resolution due to time and cost issues as described above.

In this regard, as the above-described results shown in Table 2, in modeling using the regression-type DNN, the regression of actual spatial coordinates (X, Y) could be implemented at a resolution of 0.78 mm±0.44 mm, which is an extraordinarily high level of spatial resolution which may not be achieved by a sensing device based on a conventional pattern.

In addition, since the DNN model according to the exemplary embodiment of the present invention predicts a single pressure value from 8 electric resistance signals, it is much more advantageous for securing high pressure sensitivity in a time frame (<10⁻⁴ seconds) faster than that of a conventional pressure sensing system. On the other hand, in the case of electronic skin having a conventional patterned sensor matrix, a single sensor for pressure sensing at a specific position is operated to sense a pressure value at a corresponding position. In the case of the conventional patterned electronic skin, at least a sensor should be located at every possible position of touch, which means a narrower pattern should be required to secure higher special resolution.

In the case of the electronic skin having the conventional patterned sensor matrix, the pressure value at the specific position is predicted from a single pressure versus resistance curve based on a one-to-one relationship.

Meanwhile, in the case of the DNN-driven electronic skin according to the exemplary embodiment of the present invention, it is possible to perform a so-called 8-to-1 derivation, in which one pressure value is predicted from at least eight resistance values for pressure sensing, and thus, it is possible to provide a much more sensitive pressure prediction value as compared with a conventional one-to-one derivation. In particular, it may be advantageous when data having a large amount of noise with a small gradient is used.

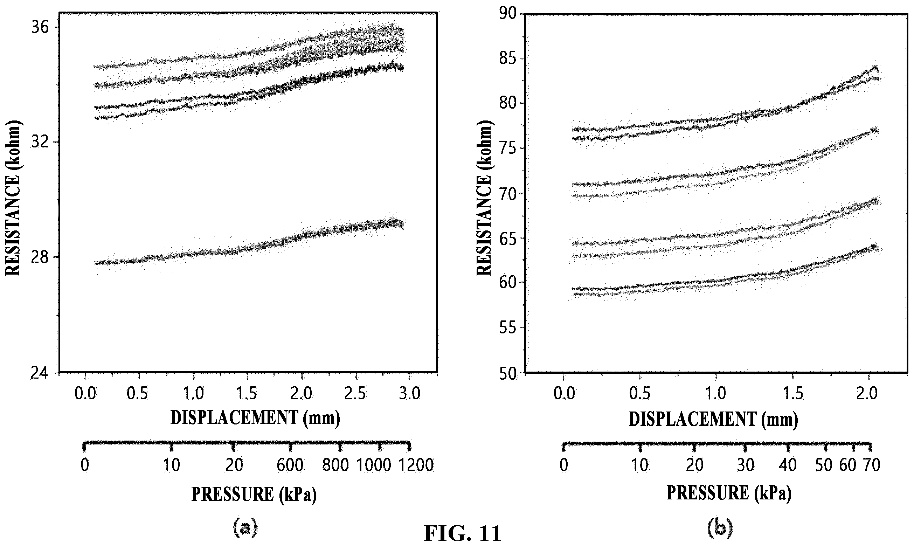

In an example, FIG. 11 shows eight electrical resistivity curves as a function of pressure at a specific sector position (fourth row and third column of a 6×6 sector matrix) of each of a thick and rigid sheet (see FIG. 11A) for high pressure measurement and a thin and smooth film for low pressure sensing (see FIG. 11B).

All pressure versus resistance curves in FIG. 11 exhibit a gradient with a low noise level which allows pressure to be reliably sensed, but sensing is possible in other cases in which there are a much more amount of noise. This is because a large number of pressure versus resistance curves (for example, 8 or 16) are used in the case of the DNN-driven electronic skin (sensor) according to the present invention.

The present invention is a result of the research performed under the Creative Materials Discovery Program, which was supported by the Ministry of Science, ICT and Future Planning and supervised by the National Research Foundation of Korea (Project No. 2105M3D1A1069705, Project name: Discovery of inorganic functional materials using heuristics-based computation).

## Claims

1. A sensing method comprising:
applying a stimulus multiple times to a random position at a material configured to generate a signal that is detectable in response to a stimulus from an outside;
performing machine learning using the signal generated through the stimulus; and
predicting a position or a degree of the stimulus applied to the material using a function derived through the machine learning.

2. The sensing method of claim 1, wherein the material configured to generate the signal that is detectable is divided into a plurality of virtual sectors, the stimulus is applied to each sector multiple times, and the machine learning is performed using a signal generated in each sector.

3. The sensing method of claim 1, wherein the machine learning is performed using coordinates of the position, to which the stimulus is applied, together with the generated signal.

4. The sensing method of any one of claims 1 to 3, wherein the material is a material on which a regular pattern is not formed.

5. The sensing method of any one of claims 1 to 3, wherein the sensing method is used for electronic skin, a touch panel, a flexible keyboard, a sign language interpretation glove, a safety diagnosis of a social infrastructure, a diagnosis of motility and motility disorder diagnosis in a gastrointestinal tract, or a sensor for a large area strain gauge.

6. The sensing method of claim 2, wherein the application of the stimulus includes applying a vertical load to each of the sectors or applying multiple, complex loads such as tearing, shearing, rubbing, pinching, etc. may be applied to a certain area spanning two or more of the sectors.

7. A sensing system comprising:
a material configured to generate a signal that is detectable in response to a stimulus from an outside;
a storage unit configured to store a function derived by performing machine learning using signals generated by applying the stimulus to the material multiple times; and
an operation unit configured to operate a position or a degree of the stimulus applied to the material using the function.

8. The sensing system of claim 7, wherein the material configured to generate the signal that is detectable is divided into a plurality of virtual sectors, the stimulus is applied to each sector multiple times, and the machine learning is performed using a signal generated in each sector.

9. The sensing system of claim 7, wherein the machine learning is performed using coordinates of the position, to which the stimulus is applied, together with the generated signal.

10. The sensing system of any one of claims 7 to 9, wherein the material is a material on which a regular pattern is not formed.

11. The sensing system of any one of claims 7 to 9, wherein the sensing system is used for electronic skin, a touch panel, a flexible keyboard, a sign language interpretation glove, a safety diagnosis of a social infrastructure, a diagnosis of motility and motility disorder diagnosis in a gastrointestinal tract, or a sensor for a large area strain gauge.
